Europäisches Patentamt

European Patent Office

Office européen des brevets

(19)

(11) Veröffentlichungsnummer : **0 446 420 B1**

(12)
# EUROPÄISCHE PATENTSCHRIFT

(45) Veröffentlichungstag der Patentschrift :
**24.11.93 Patentblatt 93/47**

(51) Int. Cl.$^5$ : **C07C 331/20**

(21) Anmeldenummer : **90120855.3**

(22) Anmeldetag : **31.10.90**

(54) **Verfahren zur Herstellung von Alkylisothiocyansäureestern.**

(30) Priorität : **16.01.90 DE 4001020**

(43) Veröffentlichungstag der Anmeldung :
**18.09.91 Patentblatt 91/38**

(45) Bekanntmachung des Hinweises auf die
Patenterteilung :
**24.11.93 Patentblatt 93/47**

(84) Benannte Vertragsstaaten :
**BE CH DE ES FR GB IT LI NL**

(56) Entgegenhaltungen :
**DE-A- 2 105 473**

(56) Entgegenhaltungen :
"**Ullmanns Encyklopädie der technischen
Chemie**", 4. neubearbeitete und erweiterte
**Auflage, 1983, Seiten 154-159, Verlag Chemie,
Weinheim, DE; "2. Organische Isothiocyanate"**

(73) Patentinhaber : **Degussa Aktiengesellschaft
Weissfrauenstrasse 9
D-60311 Frankfurt (DE)**

(72) Erfinder : **Giesselmann, Günther, Dr.
Gravenbrucherweg 17
W-6056 Heusenstamm (DE)**
Erfinder : **Günther, Kurt, Dr.
Langenselbolderweg 45
W-6455 Erlensee (DE)**

EP 0 446 420 B1

**Beschreibung**

Die Erfindung betrifft ein Verfahren zur Herstellung von Alkylisothiocyansäureestern durch Umsetzung von N-Alkyldithiocarbaminaten mit wäßrigem Wasserstoffperoxid.

Zur Herstellung von Alkylisothiocyansäureestern, auch als Alkylisothiocyanate oder Alkylsenföle bezeichnet, sind viele Verfahren bekannt geworden - siehe Ullmanns Enzyklopädie der technischen Chemie, 4. Auflage. Band 23, Seite 154-159 und DE-OS 21 05 473. Mit Ausnahme des aus der DE-PS 21 05 473 bekannten Verfahrens konnte keines der vorbekannten Verfahren Eingang in die Technik finden, weil sie entweder im Hinblick auf die Herstellung der Ausgangsstoffe zu aufwendig oder die Ausbeuten an reinen Alkylisothiocyanaten zu gering sind.

Technische Bedeutung erlangte das Verfahren der DE-OS 21 05 473 zur Herstellung von Methylisothiocyanat, das als Zwischenprodukt für organische Synthesen sowie als Nematozid und Fungizid zunehmend Verwendung findet. Nach dem Verfahren der DE-OS 21 05 473 zur Herstellung von Alkylisothiocyansäureestern der allgemeinen Formel R-N=C=S, worin R für einen niederen Alkylrest steht, wird ein Mol einer wäßrigen Lösung eines N-Alkyldithiocarbaminats der allgemeinen Formel R-NH-C(S)-SMe, worin R die vorgenannte Bedeutung hat und Me für ein Alkalimetallatom, eine Ammonium- oder Alkylammoniumgruppe stehen kann, bei einer Temperatur von 50 bis 120 °C mit mindestens 1 Mol einer 20 bis 70 gew.-%igen wäßrigen Wasserstoffperoxidlösung unter Einhaltung eines pH-Wertes von 5 bis 9 umgesetzt und der gebildete Alkylisothiocyansäureester in an sich bekannter Weise isoliert. Anspruchsgemäß wurden pro Mol Dithiocarbaminat vorzugsweise 2,3 bis 2,5 Mol Wasserstoffperoxid eingesetzt und ein pH-Wert von 6 bis 8 eingehalten; die Verwendung einer möglichst konzentrierten Dithiocarbaminatlösung wurde als besonders vorteilhaft angesehen. Als Nebenprodukte bei dieser vorbekannten Verfahrensführung entstehen bei einem Molverhältnis Dithiocarbaminat zu Wasserstoffperoxid von etwa 1 zu 2,5 pro Mol Alkylisothiocyanat ein halbes Mol Schwefel und ein halbes Mol Sulfat. Während sich diese Nebenprodukte bei der Durchführung des Verfahrens im Labormaßstab einfach abtrennen lassen, bereitet die Abtrennung des Schwefels bei der großtechnischen Herstellung Probleme: der Schwefel fällt nämlich teilweise in harten Brocken an, und zusätzlich kommt es zu Verkrustungen des Reaktors. Zur Entfernung der Brocken und Verkrustungen bedarf es häufiger und oftmals langwieriger Betriebsunterbrechungen. Hierdurch wird die Raum-Zeit-Ausbeute des Verfahrens erheblich gemindert.

Aufgabe der Erfindung ist, das Verfahren zur Herstellung von Alkylisothiocyansäureestern der Formel R-N=C=S, worin R eine Methyl- oder Ethylgruppe bedeutet, aus einer wäßrigen Lösung eines N-Alkyldithiocarbaminats der Formel R-NH-C(S)-S-Me, worin R für eine Methyl- oder Ethylgruppe und Me für ein Alkalimetallatom oder eine Ammonium- oder Alkylammoniumgruppe steht, und einer wäßrigen Wasserstoffperoxidlösung, wobei pro Mol N-Alkyldithiocarbaminat mindestens ein Mol Wasserstoffperoxid eingesetzt, die Umsetzung bei einer Temperatur von 95 bis 130 °C durchgeführt und der gebildete Alkylisothiocyansäureester in an sich bekannter Weise aus dem Reaktionsgemisch abgetrennt wird, dahingehend zu verbessern, daß dieses unter Beibehaltung einer hohen Ausbeute und hohen Reinheit des Alkylisothiocyanats mit erhöhter Raum-Zeit-Ausbeute und vermindertem Aufwand für die Reinigung des Reaktors betrieben werden kann.

Die Aufgabe wird dadurch gelöst, daß man die wäßrige N-Alkyldithiocarbaminatlösung mit einer 20 bis 40 gew.-%igen wäßrigen Wasserstoffperoxidlösung im Volumenverhältnis von 1 zu 0,8 bis 1 unter Einhaltung eines pH-Wertes von 1,5 bis 4,5 zur Reaktion bringt. Die Unteransprüche richten sich auf bevorzugte Ausführungsformen.

Durch die Kombination der das erfindungsgemäße Verfahren kennzeichnenden Merkmale entsteht bei der Reaktion überraschenderweise Natriumthiosulfat und kaum elementarer Schwefel. Wie aus den Beispielen hervorgeht, werden nur 5 bis 10 % derjenigen Menge Schwefel gebildet, welche unter den Umsetzungsbedingungen des vorbekannten Verfahrens zu erwarten waren. Hiermit bereitet die Austragung des in der wäßrigen Phase des Reaktorsumpfes suspendierten Schwefels wegen der niedrigeren Menge keine Probleme. Das erfindungsgemäße Verfahren erlaubt damit einen ungestörten Betriebsablauf sowohl bei diskontinuierlicher als auch kontinuierlicher Fahrweise, so daß bei gegebener Reaktorgröße der Umsatz in Mol/Stunde ohne Ausbeuteerniedrigung fast verdoppelt werden konnte. Erfindungsgemäß werden Methyl- und Ethylisothiocyanat in einer Reinheit von etwa 98 % und in einer Ausbeute von etwa 80 bis 95 % gewonnen.

Die Umsetzung der wäßrigen N-Alkyldithiocarbaminatlösung mit der wäßrigen Waasserstoffperoxidlösung erfolgt bei 95 bis 130 °C, vorzugsweise 100 bis 120 °C. Pro Mol N-Alkyldithiocarbaminat werden mindestens 1 bis 3 Mol, vorzugsweise 2 bis 2,5 Mol Wasserstoffperoxid eingesetzt. Die $H_2O_2$-Konzentration liegt im Bereich von 20 bis 40 Gew.-%. Wäßrige $H_2O_2$-Lösungen mit 25 bis 35 Gew.-% $H_2O_2$, wie sie durch Verdünnen von höher konzentrierten Lösungen, z. B. solchen mit 50 bis 70 Gew.-%, leicht zugänglich sind, werden bevorzugt eingesetzt. Das Verdünnen kann ggf. auch während des Einbringens der Wasserstoffperoxidlösung in den Reaktionsraum erfolgen, etwa durch gleichzeitiges Versprühen einer konzentrierteren Wasserstoffperoxidlösung und Wasser, so daß eine anspruchsgemäße Konzentration resultiert.

Die Konzentration der wäßrigen N-Alkyldithiocarbaminatlösung sollte vorzugsweise im Bereich von 30 bis 45 Gew.-% liegen. Der pH-Wert der Lösung wird auf 9,5 bis 12,0 eingestellt. Die Herstellung solcher Lösungen erfolgt in an sich bekannter Weise; die vorzugsweise zu verwendende Lösung des Natrium-N-alkyldithiocarbaminats ist aus dem Alkylamin, Schwefelkohlenstoff und Natronlauge zugänglich.

Die Umsetzung der beiden Lösungen erfolgt im beanspruchten Volumenverhältnis so, daß in der wäßrigen Phase des Reaktionsgemisches ein pH-Wert von 1,5 bis 4,5, vorzugsweise 2,0 bis 4,0 eingehalten wird. Im vorbekannten Verfahren der DE-PS 21 05 473, wonach der pH-Wert zwischen 5 und 9 liegen sollte, war zwar kein Volumenverhältnis der N-Alkyldithiocarbaminatlösung zur Wasserstoffperoxidlösung genannt, aus den Beispielen ergibt sich jedoch ein solches im Bereich von 1 zu 0,45 bis 0,75, das damit außerhalb des nun beanspruchten Verfahrens liegt.

Die miteinander umzusetzenden Lösungen können durch Zusammenführen aus getrennten Zuleitungen in einem Reaktor zur Reaktion gebracht werden. Bei der Reaktionstemperatur von 95 bis 130 °c wird das Alkylisothiocyanat im allgemeinen unmittelbar nach seiner Bildung von der die Nebenprodukte enthaltenden wäßrigen Phase dampfförmig abgetrennt. Der sich durch geringfügige Zersetzung von Dithiocarbaminat bildende Schwefelkohlenstoff wird durch Destillation des rohen Alkylisothiocyanats quantitativ zurückgewonnen und kann der Herstellung des Dithiocarbaminats zugeführt werden.

Die Isolierung der Alkylisothiocyansäureester erfolgt in an sich bekannter Weise, z. B. durch Extraktion mittels z. B. Ketonen, aliphatischen oder aromatischen Kohlenwasserstoffen, Methylenchlorid, Chloroform, Tetrachlorkohlenstoff, Schwefelkohlenstoff, Ethern, Estern oder Nitrobenzol. Vorteilhafter, insbesondere bei der kontinuierlichen Führung des Verfahrens, ist jedoch die Abtrennung durch Azeotrop-Destillation, die auch unter Zusatz von Wasserdampf erfolgen kann. Die Ausbeute läßt sich durch diese Maßnahme signifikant erhöhen. Auch die sehr hohe Wärmetönung der Reaktion des Dithiocarbaminates mit dem Wasserstoffperoxid kann ausgenutzt werden. Diese ist so groß, daß für die Destillation des Isothiocyansäureesters keine zusätzliche Energie benötigt wird.

Gemäß einer besonders bevorzugten Ausführungsform werden die wäßrige N-Alkyldithiocarbaminatlösung und die wäßrige Wasserstoffperoxidlösung in Form von Tröpfchen, insbesondere feinst verteilten Tröpfchen miteinander zur Reaktion gebracht. Übliche Vorrichtungen zur Überführung einer Flüssigkeit in Tröpfchenform sind geeignet. Vorzugsweise erreicht man eine feine Verteilung der Komponenten durch Verwendung von Düsen, wobei Ein- oder Mehrstoffdüsen eingesetzt werden können. Eine oder mehrere Düsen können im Reaktor, etwa im Deckel und/oder im oberen Teil der Wandung angeordnet sein.

Je nach Reaktorgröße wird der Sprühwinkel der Düsen so ausgelegt, daß die Hauptreaktion im Zentrum des oberen Drittels des Reaktors stattfindet, um Kondensation an der Innenwand zu vermeiden. Der Reaktor wird vor Reaktionsbeginn, entsprechend seinem Volumen zu etwa 1/8 bis etwa 1/3 mit Wasser gefüllt. Bei kontinuierlicher Fahrweise werden die sich bildenden Nebenprodukte fortlaufend aus dem Reaktor entfernt und, soweit erforderlich, durch Wasser ersetzt.

Durch die Beispiele wird die Erfindung weiter offenbart.

Beispiel 1

Ein mit einer Sprüheinrichtung für die miteinander umzusetzenden Lösungen, einem Rührer, einem Brüdenabgang, Sumpfablaß und pH- und Temperaturmeßeinrichtungen ausgestatteter heiz- und kühlbarer Reaktor wird zu 15 % seines Volumens mit Wasser befüllt Nach Aufheizen der wäßrigen Vorlage auf etwa 98 °C werden innerhalb 90 Minuten 3 Mol (770 ml) einer in bekannter Weise aus Methylamin, Schwefelkohlenstoff und Natriumhydroxid hergestellten und auf pH 10 eingestellten wäßrigen 42 gew.-%igen N-Methyldithiocarbaminatlösung mit 7,5 Mol (700 ml) einer 32,5 gew.-%igen wäßrigen Wasserstoffperoxidlösung zur Reaktion gebracht. Hierzu werden die wäßrige N-Methyldithiocarbaminatlösung und die wäßrige Wasserstoffperoxidlösung im Volumenverhältnis 1 : 0,91 (Molverhältnis 1 : 2,5) unter Verwendung einer Düse mit einem Innendurchmesser von 2 mm zusammengeführt und feinst verteilt in den Reaktor versprüht. Das gebildete Methylisothiocyanat wird umittelbar nach seiner Entstehung abdestilliert. Während der Umsetzung werden der pH-Wert der flüssigen Phase (Reaktorsumpf) und die Temperatur am Brüdenabgang verfolgt - siehe Tabelle 1.

**Tabelle 1**

| Zeit (Min) | pH-Wert | Temperatur (°C) |
|---|---|---|
| 15 | 2,1 | 100,6 |
| 30 | 2,4 | 100,1 |
| 45 | 2,6 | 100,7 |
| 60 | 2,5 | 101,6 |
| 75 | 2,5 | 102,9 |
| 90 | 2,0 | 102,8 |

Die Wärmetönung der Reaktion Dithiocarbaminat / Wasserstoffperoxid ist so groß, daß für die Destillation des Methylisothiocyanats keine zusätzliche Energie benötigt wird. Damit sich das Methylisothiocyanat flüssig abscheidet, wird die Temperatur der Kühler auf 35 bis 38 °c eingestellt. In dem gleichzeitig mit überdestillierenden Wasser sind noch etwa 5 bis 7 % Methylisothiocyanat gelöst, die durch Extraktion mit n-Hexan abgetrennt werden. (Diese Abtrennung kann alternativ auch durch Andestillation der Wasserphase erfolgen.)

Man erhält 2,6 Mol 98 %iges Methylisothiocyanat, entsprechend einer Ausbeute von 87 %; in der wäßrigen Phase (Reaktorsumpf) sind 4 g Schwefel, also nur 8,3 % der bei der vorbekannten Verfahrensführung zu erwarten den Menge, suspendiert.

Beispiel 2

Analog Beispiel 1 werden miteinander umgesetzt: 770 ml (3 Mol) auf pH 11,0 eingestellte 42 gew.-%ige wäßrige N-Methyldithiocarbaminatlösung mit 650 ml (7,5 Mol) einer 35 gew.-%igen wäßrigen Wasserstoffperoxidlösung - Volumenverhältnis der Lösungen 1 : 0,84, Molverhältnis der Reaktanden 1 : 2,5, Der pH-Wert der flüssigen Phase und die Temperatur am Brüdenabgang während der Umsetzung folgen aus Tabelle 2.

**Tabelle 2**

| Zeit (Min) | pH-Wert | Temperatur (°C) |
|---|---|---|
| 15 | 3,9 | 99 |
| 30 | 4,2 | 99 |
| 45 | 3,6 | 100 |
| 60 | 3,6 | 100 |
| 75 | 3,6 | 101 |
| 90 | 3,7 | 101 |

Man erhält 2,51 Mol 98 %iges Methylisothiocyanat, entsprechend einer Ausbeute von 84 %; die wäßrige Phase enthält 5 g Schwefel (10,4 % der theor. möglichen Menge).

Beispiel 3

Analog Beispiel 1 werden miteinander umgesetzt: 770 ml (3 Mol) auf pH 10 eingestellte 42 gew.-%ige N-Methyldithiocarbaminatlösung mit 644 ml (6,9 Mol) einer 32,5 gew.-%igen wäßrigen Wasserstoffperoxidlösung - Volumenverhältnis der Lösungen 1 : 0,84, Molverhältnis der Reaktanden 1 ; 2,3, Der pH-Wert der flüssigen Phase und die Temperatur am Brüdenabgang während der Umsetzung folgen aus Tabelle 3.

Tabelle 3

| Zeit (Min) | pH-Wert | Temperatur (°C) |
|---|---|---|
| 15 | 4,5 | 99 |
| 30 | 4,2 | 100 |
| 45 | 4,4 | 101 |
| 60 | 4,3 | 101,5 |
| 75 | 4,3 | 102 |
| 90 | 4,3 | 103 |

Man erhält 2,45 Mol 98 %iges Methylisothiocyanat, entsprechend einer Ausbeute von 82 %; die wäßrige Phase enthält 3,7 g Schwefel (7,7 % der theor. möglichen Menge).

Beispiel 4

Analog Beispiel 1 werden miteinander umgesetzt: 770 ml (3 Mol auf pH 10 eingestellte 42 gew.-%ige wäßrige N-Methyldithiocarbaminatlösung mit 760 ml (7,5 Mol) einer 30 gew. -%igen wäßrigen Wasserstoffperoxidlösung - Volumenverhältnis der Lösungen 1 : 0,99, Molverhältnis der Reaktanden 1 : 2,5. Der pH-Wert und die Temperatur während der Umsetzung folgen aus Tabelle 4.

Tabelle 4

| Zeit (Min) | pH-Wert | Temperatur (°C) |
|---|---|---|
| 15 | 4,0 | 100 |
| 30 | 3,5 | 101 |
| 45 | 3,2 | 101,5 |
| 60 | 3,2 | 101,5 |
| 75 | 2,5 | 102 |
| 90 | 2,8 | 102,5 |

Man erhält etwa 2,45 Mol 98 %iges Methylisothiocyanat, entsprechend einer Ausbeute von 82 %; die wäßrige Phase enthält 3 g Schwefel (6,2 % der theor. möglichen Menge).

Beispiel 5

Analog Beispiel 1 werden miteinander umgesetzt: 770 ml (3 Mol) auf pH 10 eingestellte 42 gew.-%ige wäßrige N-Methyldithiocarbaminatlösung mit 700 ml (7,5 Mol) einer 32,5 gew.-%igen wäßrigen Wasserstoffperoxidlösung - Volumenverhältnis der Lösungen 1 : 0,91, Molverhältnis der Reaktanden 1 : 2,5, Während der Umsetzung werden zusätzlich 350 g Wasserdampf in den Reaktionsraum eingeblasen, Der pH-Wert und die Temperatur während der 90-minütigen Umsetzung folgen aus Tabelle 5.

Tabelle 5

| Zeit (Min) | pH-Wert | Temperatur ($^{\circ}$C) |
|---|---|---|
| 15 | 2,2 | 100,5 |
| 30 | 2,3 | 100,6 |
| 45 | 2,0 | 101,2 |
| 60 | 1,9 | 101,8 |
| 75 | 2,1 | 102,3 |
| 90 | 2,0 | 102,5 |

Man erhält 2,7 Mol 98 %iges Methylisothiocyanat, entsprechend einer Ausbeute von 90 %; in der wäßrigen Phase sind 3,5 g Schwefel (7 % der theor. möglichen Menge) suspendiert.

Beispiel 6

515 ml (1,5 Mol 36 gew.-%ige, auf pH 10 eingestellte wäßrige N-Ethyldithiocarbaminatlösung, hergestellt in bekannter Weise aus Ethylamin, Schwefelkohlenstoff und Natronlauge, werden innerhalb 90 Minuten mit 410 ml (3,75 Mol) 31 gew.-%iger wäßriger Wasserstoffperoxidlösung analog Beispiel 1 verdüst. Das Volumenverhältnis der N-Ethyldithiocarbaminat- zur Wasserstoffperoxidlösung entspricht 1 : 0,8, das Molverhältnis der Reaktanden 1 : 2,5. Der pH-Wert der flüssigen Phase (Reaktorsumpf) und die Temperatur am Brüdenabgang während der Umsetzung sind der Tabelle 6 zu entnehmen.

Tabelle 6

| Zeit (Min) | pH-Wert | Temperatur ($^{\circ}$C) Übergang |
|---|---|---|
| 15 | 3,2 | 100,2 |
| 30 | 3,0 | 100,7 |
| 45 | 2,9 | 101 |
| 60 | 3,0 | 101,6 |
| 75 | 2,8 | 102 |
| 90 | 2,9 | 102,3 |

Es werden 1,38 Mol 98 %iges Ethylisothiocyanat, entsprechend einer Ausbeute von 92,4 %, und 2 g Schwefel (8,3 % der theor. möglichen Menge) gebildet.

**Patentansprüche**

1. Verfahren zur Herstellung von Alkylisothiocyansäureestern der Formel R-N=C=S, worin R eine Methyl- oder Ethylgruppe bedeutet, aus einer wäßrigen Lösung eines N-Alkyldithiocarbaminats der Formel R-NH-C(S)-S-Me, worin R für eine Methyl- oder Ethylgruppe und Me für ein Alkalimetallatom oder eine Ammonium- oder Alkylammoniumgruppe steht, und einer wäßrigen Wasserstoffperoxidlösung, wobei pro Mol N-Alkyldithiocarbaminat mindestens ein Mol Wasserstoffperoxid eingesetzt, die Umsetzung bei einer Temperatur von 95 bis 130 °C durchgeführt und der gebildete Alkylisothiocyansäureester in an sich bekannter Weise aus dem Reaktionsgemisch abgetrennt wird,

dadurch gekennzeichnet,
daß man die wäßrige N-Alkyldithiocarbaminatlösung mit einer 20 bis 40 gew.-%igen wäßrigen Wasserstoffperoxidlösung im Volumenverhältnis von 1 zu 0,8 bis 1 unter Einhaltung eines pH-Wertes von 1,5 bis 4,5 zur Reaktion bringt.

2. Verfahren nach Anspruch 1,
dadurch gekennzeichnet,
daß man eine 25 bis 35 gew.-%ige wäßrige Wasserstoffperoxidlösung einsetzt.

3. Verfahren nach Anspruch 1 oder 2,
dadurch gekennzeichnet,
daß man eine 30 bis 45 gew.-%ige, vorzugsweise auf einen pH-Wert von 9,5 bis 12 eingestellte wäßrige N-Alkyldithiocarbaminatlösung einsetzt.

4. Verfahren nach einem oder mehreren der Ansprüche 1 bis 3,
dadurch gekennzeichnet,
daß man die Umsetzung bei einer Temperatur von 100 bis 120 °C durchführt.

5. Verfahren nach einem oder mehreren der Ansprüche 1 bis 4,
dadurch gekennzeichnet,
daß man pro Mol N-Alkyldithiocarbaminat 2 bis 2,5 Mol Wasserstoffperoxid einsetzt.

6. Verfahren nach einem oder mehreren der Ansprüche 1 bis 5,
dadurch gekennzeichnet,
daß man im Reaktionsgemisch einen pH-Wert im Bereich von 2,0 bis 4,0 einhält.

7. Verfahren nach einem oder mehreren der Ansprüche 1 bis 6,
dadurch gekennzeichnet,
daß man die wäßrige N-Alkyldithiocarbaminatlösung und die wäßrige Wasserstoffperoxidlösung in Form von Tröpfchen miteinander zur Reaktion bringt.

8. Verfahren nach Anspruch 7,
dadurch gekennzeichnet,
daß man die Umsetzung im wesentlichen in feinen Tröpfchen, welche durch Versprühen der Lösungen unter Verwendung einer oder mehrerer Ein- und/oder Zweistoffdüsen erhalten werden, durchführt und den dabei entstehenden Alkylisothiocyansäureester dampfförmig aus dem Reaktionsraum abzieht.

9. Verfahren nach einem oder mehreren der Ansprüche 1 bis 8,
dadurch gekennzeichnet,
daß man das Verfahren kontinuierlich durchführt, indem man den gebildeten Alkylisothiocyansäureester und unter den Reaktionsbedingungen flüchtige Nebenprodukte unmittelbar nach ihrer Entstehung dampfförmig und die nichtflüchtigen Nebenprodukte mit der wäßrigen Phase aus dem Reaktionsraum abtrennt und, soweit erforderlich, das Flüssigkeitsniveau im Reaktor durch Zugabe von Wasser aufrechterhält.

10. Verfahren nach einem oder mehreren der Ansprüche 1 bis 9,
dadurch gekennzeichnet,
daß man während der Reaktion in den Reaktor zusätzlich Wasserdampf einbläst.

## Claims

1. A process for the preparation of alkyl isothyocyanic acid esters of the formula R-N=C=S, wherein R denotes a methyl or ethyl group, from an aqueous solution of an N-alkyl-dithiocarbaminate of the formula R-NH-C(S)-S-Me, wherein R stands for a methyl or ethyl group and Me for an alkali metal atom or an ammonium or alkyl ammonium group, and an aqueous hydrogen peroxide solution, at least one mol of hydrogen peroxide being used per mol of N-alkyl-dithiocarbaminate, the reaction being carried out at a temperature of from 95 to 130°C and the alkyl isothiocyanic acid ester formed being separated from the reaction mixture in known manner, characterised in that the aqueous N-alkyl-dithiocarbaminate solution is reacted with a 20 to 40% by weight aqueous hydrogen peroxide solution in a volumetric ratio of from 1:1 to 0.8:1 while a pH of from 1.5 to 4.5 is maintained.

2. A process according to claim 1, characterised in that a 25 to 35% by weight aqueous hydrogen peroxide solution is used.

3. A process according to claim 1 or claim 2, characterised in that a 30 to 45% by weight aqueous N-alkyl-dithiocarbaminate solution preferably adjusted to a pH of from 9.5 to 12 is used.

4. A process according to one or more of claims 1 to 3, characterised in that the reaction is carried out at a temperature of from 100 to 120°C.

5. A process according to one or more of claims 1 to 4, characterised in that from 2 to 2.5 mol of hydrogen peroxide are used per mol of N-alkyl-dithiocarbaminate.

6. A process according to one or more of claims 1 to 5, characterised in that a pH in the range of from 2.0 to 4.0 is maintained in the reaction mixture.

7. A process according to one or more of claims 1 to 6, characterised in that the aqueous N-alkyl-dithiocarbaminate solution and the aqueous hydrogen peroxide solution are reacted together in the form of droplets.

8. A process acording to claim 7, characterised in that the reaction is carried out substantially in fine droplets which are obtained by atomising the solutions, using one or more one-material or two-material nozzles and the resulting alkyl-isothiocyanic acid ester is withdrawn from the reaction chamber in vapour form.

9. A process according to one or more of claims 1 to 8, characterised in that the process is carried out continuously by removing the alkyl-isothiocyanic acid ester formed and the by-products which are volatile under the reaction conditions from the reaction chamber in vapour form immediately after their formation and removing the nonvolatile by-products from the reaction chamber with the aqueous phase and, if necessary, maintaining the liquid level in the reactor by the addition of water.

10. A process according to one or more of claims 1 to 9, characterised in that additional steam is injected into the reactor during the reaction.

## Revendications

1. Procédé d'obtention d'esters d'acide alcoylisothioacyanique de formule R - N = C = S dans laquelle R signifie groupe méthyle ou éthyle, à partir d'une solution aqueuse d'un N-alcoyldithiocarbaminate de formule R-NH-C(S)-S-Me dans laquelle R représente un groupe méthyle ou éthyle et Me représente un atome de métal alcalin, ou un groupe ammonium ou alcoylammonium, et d'une solution aqueuse de peroxyde d'hydrogène, procédé dans lequel on met en oeuvre par mol de N-alcoyldithiocarbaminate au moins une mol de peroxyde d'hydrogène, on effectue la réaction à une température allant de 95° à 130°C, et on sépare l'ester d'acide alcoylisothiocyanique formé du mélange réactionnel d'une manière connue en soi, caractérisé en ce que l'on amène en réaction la solution aqueuse de N-alcoyldithiocarbaminate avec une solution aqueuse de peroxyde d'hydrogène de 20 à 40 % en poids dans un rapport volumique de 1 à 0,8 jusqu'à 1, tout en maintenant une valeur de pH allant de 1,5 à 4,5.

2. Procédé selon la revendication 1, caractérisé en ce que l'on met en oeuvre une solution de peroxyde d'hydrogène de 25 à 35 % en poids.

3. Procédé selon la revendication 1 ou 2, caractérisé en ce que l'on met en oeuvre une solution aqueuse de N-alcoyldithiocarbaminate de 30 à 45 % en poids, de préférence ajustée à une valeur de pH allant de 9,5 à 12.

4. Procédé selon l'une ou plusieurs des revendications 1 à 3, caractérisé en ce que l'on exécute la réaction à une température de 100 à 120°C.

5. Procédé selon l'une ou plusieurs des revendications 1 à 4, caractérisé en ce que l'on met en oeuvre par mol de N-alcoyldithiocarbaminate de 2 à 2,5 mol de peroxyde d'hydrogène.

6. Procédé selon l'une ou plusieurs des revendications 1 à 5, caractérisé en ce que l'on maintient dans le mélange réactionnel une valeur de pH dans la plage de 2,0 à 4,0.

7. Procédé selon l'une ou plusieurs des revendications 1 à 6, caractérisé en ce que l'on amène en réaction l'une avec l'autre, la solution aqueuse de N-alcoyldithiocarbaminate et la solution aqueuse de peroxyde d'hydrogène sous forme de gouttelettes.

8. Procédé selon la revendication 7, caractérisé en ce que l'on exécute la réaction essentiellement sous forme de fines gouttelettes qui sont obtenues par pulvérisation des solutions en utilisant un ou plusieurs injecteurs pour une et/ou deux substances, et que l'on soutire l'ester d'acide alcoylisothiocyanique qui est ainsi formé, sous forme de vapeur de l'espace réactionnel.

9. Procédé selon l'une ou plusieurs revendications 1 à 8, caractérisé en ce que l'on effectue le procédé en continu, en séparant l'ester d'acide alcoylisothiocyanique formé et les produits secondaires volatils dans les conditions de la réaction immédiatement après leur formation sous forme de vapeur, et les produits secondaires non volatils avec la phase aqueuse, de l'espace réactionnel et pour autant que cela soit nécessaire, en maintenant le niveau de liquide dans le réacteur par addition d'eau.

10. Procédé selon l'une ou plusieurs des revendications 1 à 9, caractérisé en ce que l'on insuffle pendant la réaction de la vapeur d'eau en supplément dans le réacteur.